Europäisches Patentamt

European Patent Office

Office européen des brevets

(11) Numéro de publication: **0 196 932**
**A1**

## (12) DEMANDE DE BREVET EUROPEEN

(21) Numéro de dépôt: **86400317.3**

(22) Date de dépôt: **14.02.86**

(51) Int. Cl.⁴: **A 61 K 31/80**
**A 61 K 9/24, A 61 K 9/54**

(30) Priorité: **22.02.85 FR 8502623**

(43) Date de publication de la demande:
**08.10.86 Bulletin 86/41**

(84) Etats contractants désignés:
**AT BE CH DE GB IT LI LU NL SE**

(71) Demandeur: **Grimberg, Georges Serge**
**123 rue de l'Université**
**F-75007 Paris(FR)**

(72) Inventeur: **Grimberg, Georges Serge**
**123 rue de l'Université**
**F-75007 Paris(FR)**

(74) Mandataire: **Madeuf, Claude Alexandre Jean et al,**
**CABINET MADEUF 3, avenue Bugeaud**
**F-75116 Paris(FR)**

(54) **Médicament non absorbable utilisé sous forme gastro-soluble et gastro-résistante.**

(57) Médicament gastro-intestinal non absorbable mais pouvant être soluble destiné à soigner les deux niveaux du tube digestif caractérisé en ce qu'il est présenté sous une forme galénique pharmaceutique gastro-résistante associée à une forme galénique pharmaceutique gastro-soluble.

EP 0 196 932 A1

Croydon Printing Company Ltd

**0196932**

Médicament gastro-intestinal non absorbable destiné à soigner les deux niveaux du tube digestif et présenté sous une forme galénique gastro-soluble et gastro-résistante.

Certaines maladies du tube digestif sont difficiles à localiser. Les symptômes sont larges et, très souvent, l'estomac et le tube digestif sont concernés.

Si l'on considère les médicaments non absorbés par l'organisme, c'est-à-dire les médicaments avalés et évacués par le tube digestif sans pénétration dans le sang, leur action aux différents étages du tube digestif ne peut avoir lieu que si elle n'est pas épuisée par l'étage précédent.

La présente invention a donc pour objet de proposer un médicament non absorbable par l'organisme et permettant de traiter chacun des niveaux du tube digestif du fait que le premier niveau reçoit une certaine quantité du médicament qui se délite très rapidement puis le deuxième niveau reçoit une autre quantité du médicament qui est protégé soit par une gélule soit par un excipient gastro-résistant.

Conformément à l'invention, le médicament gastro-intestinal non absorbable mais pouvant être soluble destiné à soigner les deux niveaux du tube digestif est caractérisé en ce qu'il est présenté sous une forme galénique pharmaceutique gastro-résistante associée à une forme galénique pharmaceutique gastro-soluble.

Diverses autres caractéristiques de l'invention ressortent d'ailleurs de la description détaillée qui suit et des exemples indiqués pour étayer l'invention.

Si l'on prend, par exemple, du Siméthicone, on constate que si l'on donne à l'organisme 100 mg de Siméthicone dans une forme galénique pharmaceutique gastro-soluble, l'action sur les malades est nettement inférieure à celle que donnent 50 mg de Siméthicone dans une forme galénique pharmaceutique gastro-soluble plus 50 mg de Siméthicone dans une forme galénique pharmaceutique gastro-résistante ; ces deux capsules étant prises en même temps.

Cette supériorité a été vérifiée sur des malades présentant différents symptômes tels que : météorisme, émission de gaz par l'anus, éructations, lenteurs de digestion, somnolence post-prandiale, impression de gêne après les repas.

Chacun de ces symptômes était coté de 0 à 3 selon son intensité : inexistant : 0 ; intensité légère : 1 ; moyenne : 2 ; marquée : 3.

Cette quantification était faite par le malade et vérifiée par l'expérimentateur.

Pour le contrôle il a été administré deux sortes de produits :

Produit A : forme galénique pharmaceutique gastro-soluble de Siméthicone,

Produit B : forme galénique pharmaceutique gastro-résistant de Siméthicone.

Les malades divisés en deux groupes (I et II) de 50 malades chacun ont pris :

Groupe I : 3 fois par jour et à chaque fois, 2 fois 50 mg de produit A,

Groupe II : 3 fois par jour et à chaque fois, 1 fois 50 mg de produit A, plus une fois 50 mg de produit B.

3         **0196932**

La durée du traitement a été de 15 jours de To à T15.

La différence de score pour chaque malade entre le temps To et le temps T15 a permis d'étudier les deux groupes de malades.

Il en résulte une différence statistiquement significative entre les deux groupes.

Le groupe II, c'est-à-dire le groupe qui prend le produit A + le produit B est supérieur au groupe I, c'est-à-dire le groupe qui prend le produit A + le produit A.

Le problème est exactement le même avec du charbon médicinal ou avec certains enzymes.

0196932

Revendications

1 - Médicament gastro-intestinal non absorbable mais pouvant être soluble destiné à soigner les deux niveaux du tube digestif, caractérisé en ce qu'il est présenté sous une forme galénique pharmaceutique gastro-résistante associée à une forme galénique pharmaceutique gastro-soluble.

2 - Médicament selon la revendication 1, caractérisé en ce qu'il comprend obligatoirement la forme gastro-soluble et la forme gastro-résistante.

Office européen
des brevets

**RAPPORT DE RECHERCHE EUROPEENNE**

0196932
Numero de la demande

EP  86 40 0317

## DOCUMENTS CONSIDERES COMME PERTINENTS

| Catégorie | Citation du document avec indication. en cas de besoin. des parties pertinentes | Revendication concernee | CLASSEMENT DE LA DEMANDE (Int Cl 4) |
|---|---|---|---|
| X,Y | FR-A-2 390 953  (GRIMBERG) <br> * En entier et en particulier page 2, exemples 2,3; page 3, lignes 27-29 * | 1,2 | A 61 K   31/80 <br> A 61 K    9/24 <br> A 61 K    9/54 |
| | --- | | |
| X | AU-B- 407 324 (SMITH KLINE & FRENCH) <br> * Page 3, ligne 1 - page 5, ligne 11; revendication 6 * | 1,2 | |
| | --- | | |
| Y | FR-A-2 350 836  (SHIONOGI) <br> * Revendications 1,2,8-13 * | 1,2 | |
| | ----- | | |

DOMAINES TECHNIQUES RECHERCHES (Int Cl 4)

A 61 K

Le présent rapport de recherche a été établi pour toutes les revendications

| Lieu de la recherche <br> LA HAYE | Date d'achèvement de la recherche <br> 09-05-1986 | Examinateur <br> BENZ K.F. |
|---|---|---|